# EUROPÄISCHE PATENTSCHRIFT

(11) **EP 2 848 189 B1**
(45) Veröffentlichungstag und Bekanntmachung des Hinweises auf die Patenterteilung: **08.03.2017**
(21) Anmeldenummer: 14184546.1
(22) Anmeldetag: 12.09.2014
(51) Int. Cl.: A61B 1/00, A61B 1/005, A61M 25/01

(54) **Endoskopisches System**
Endoscopic system
Système endoscopic

(30) Priorität: 17.09.2013 DE 102013110227
(43) Veröffentlichungstag der Anmeldung: 18.03.2015
(73) Patentinhaber: Karl Storz GmbH & Co. KG, 78532 Tuttlingen (DE)
(72) Erfinder: Ayrenschmalz, Robert, 78532 Tuttlingen (DE); Wimmer, Viktor, 78532 Tuttlingen (DE)

(56) Entgegenhaltungen:
- EP-A1- 1 987 789
- EP-A1- 2 332 459
- US-A1- 2003 018 237
- US-A1- 2007 045 504
- US-A1- 2012 232 476

## Beschreibung

Die vorliegende Erfindung betrifft ein endoskopisches System, insbesondere ein endoskopisches System für die Koloskopie, nach dem Oberbegriff des Anspruchs 1.

Bei einer endoskopischen Untersuchung eines körperinneren Hohlraums, etwa eines Hohlorgans, mit Hilfe eines flexiblen Endoskops wird ein langerstreckter flexibler Einführschaft des Endoskops durch eine natürliche oder künstlich geschaffene Körperöffnung in den zu untersuchenden körperinneren Hohlraum eingeführt. Am distalen Ende des Einführschafts ist eine Optik zur Erzeugung eines endoskopischen Bildes angeordnet. Zur Aufnahme und Weiterleitung des endoskopischen Bildes vom distalen (d.h. beobachterfernen) Endbereich des Einführschafts zu einem Kontrollteil, das in einem proximalen (d.h. beobachternahen) Endbereich des Schafts angeordnet ist, kann beispielsweise innerhalb des Schafts ein geordnetes Bündel von Lichtleitfasern verlaufen; in diesem Fall ist üblicherweise im oder am Kontrollteil eine endoskopische Kamera angeordnet, das Kontrollteil kann aber auch ein Okular für einen visuellen Einblick aufweisen. Andererseits kann auch ein elektronischer Bildaufnehmer im distalen Endbereich des Einführschafts angeordnet sein, dessen Signale über innerhalb des Schafts verlaufende elektrische Leitungen zum Kontrollteil übertragen werden. Das Kontrollteil weist einen Videoanschluss zur Verbindung mit einer externen Einrichtung zur Auswertung, Anzeige und Speicherung des aufgenommenen endoskopischen Bildes auf. Ferner kann innerhalb des Schafts ein Lichtleitsystem angeordnet sein, um über einen am Kontrollteil angeordneten Anschluss von einer externen Lichtquelle eingekoppeltes Beleuchtungslicht an das distale Ende des Endoskops zu transportieren, wo es zur Beleuchtung des Hohlraums genutzt wird.

Häufig ist es notwendig, das distale Ende des Schafts abwinkeln zu können, um die Einführung des Endoskops durch einen nicht geradlinig verlaufenden Kanal zu erleichtern, um die Spitze des Endoskopschafts innerhalb des Hohlraums in einer seitlichen Richtung bewegen zu können und um die Blickrichtung der in der Endoskopspitze angeordneten Optik verändern zu können. Hierfür weist der Einführschaft einen steuerbaren Abschnitt, insbesondere einen steuerbaren Endabschnitt, auf, der aktiv in eine gewünschte Richtung abgewinkelt werden und hierfür vom proximalen Endbereich des Endoskops her gesteuert werden kann. Zur Steuerung der Abwinklung des distalen Endbereichs des Einführschafts sind üblicherweise am Kontrollteil entsprechende Bedienelemente, beispielsweise Drehräder zur Betätigung von innerhalb des Endoskopschafts verlaufenden, die Abwinkelung bewirkenden Seilzügen, angeordnet. Das Kontrollteil ist hierbei als Handstück ausgebildet, das neben den Drehrädern zur Steuerung der Ablenkung des distalen Endbereichs des Einführschafts weitere Bedienelemente, wie etwa Tasten zur Bedienung von Spül- und Saugventilen, umfasst. An dem als Handstück ausgebildeten Kontrollteil setzt vorzugsweise ein Versorgungskabel an, das einen Beleuchtungslichtleiter, ein Videokabel und Saug- und Spülleitungen umfasst und durch welches diese zu den entsprechenden Versorgungseinrichtungen geführt werden.

Weiterhin ist es bekannt, dass der Schaft des flexiblen Endoskops einen oder mehrere Arbeitskanäle zur Durchführung endoskopischer Arbeitsinstrumente aufweist, mit denen beispielsweise eine Probenentnahme oder auch chirurgische Manipulationen innerhalb des Hohlraums vorgenommen werden können. Am Kontrollteil oder im Bereich des Kontrollteils sind in diesem Fall Öffnungen zur Einführung der endoskopischen Arbeitsinstrumente angeordnet.

Bei einer endoskopischen Untersuchung oder Operation mit Hilfe eines derartigen flexiblen Endoskops ist häufig eine abwechselnde oder gleichzeitige Ausführung unterschiedlicher Bewegungen des Endoskopschafts notwendig. So ist zum Einführen des Einführschafts in einen gekrümmten Zugangsweg neben dem Vorschieben des Schafts eine der jeweiligen Krümmung des Zugangswegs entsprechende Abwinkelung des distalen Endbereichs des Einführschafts notwendig sowie gegebenenfalls eine Drehung des Einführschafts um seine Längsachse. Dies gilt insbesondere bei einem mehrfach in unterschiedliche Richtungen gekrümmten Zugangsweg, wie etwa dem unteren und dem oberen Verdauungstrakt. Hierbei ist nicht nur eine mehrfache Änderung der Abwinkelung des distalen Endbereichs des Einführschafts und eine mehrfache Drehung des Schafts um seine Längsachse erforderlich, sondern die Vorschubbewegung muss gelegentlich unterbrochen und der Schaft ein kurzes Stück zurückgezogen werden, um einen optimalen Durchgang durch den gekrümmten Verdauungstrakt zu erreichen und eine übermäßige Belastung der Darmwände zu vermeiden. Die Ausführung einer derartigen Vorschubbewegung erfordert ein erhebliches Maß an Übung des Operateurs und ist trotzdem für den Patienten häufig schmerzhaft und mit Risiken verbunden.

Aus EP 1 987 789 A1 ist ein Endoskopsystem bekannt, das ein flexibles Endoskop mit einem langerstreckten Einführungsteil und ein medizinisches Arbeitsinstrument mit einem in einem distalen Bereich angeordneten Behandlungsteil und einem langen Schaft, der in einen Kanal in dem Einführungsteil eingeführt wird, umfasst. Das Endoskopsystem umfasst ferner einen Operationsbedienungsapparat zur Eingabe von Befehlen zur Bedienung des medizinischen Arbeitsinstruments, einen ersten Antriebsapparat zum Betreiben des Behandlungsteils und einen zweiten Antriebsapparat, der den Schaft des medizinischen Arbeitsinstruments vor- und rückwärts bewegt. Der Operationsbedienungsapparat umfasst einen Bedienhebel, mit dem die motorischen Antriebe des medizinischen Arbeitsinstruments betätigt werden können. Der Operationsbedienungsapparat wird hierfür über den Schaft des Endoskops geschoben. Nachdem die motorischen Antriebe zum Betätigen des Behandlungsteils und zum Vor- und Zurückschieben des Instruments an dem Endoskop angebracht worden sind, wird der Schaft des Endoskops in eine Körparhöhle eines Patienten eingeführt. Zur Entnahme einer Gewebeprobe in der Körperhöhle kann der Chirurg durch Betätigung des Bedienhebels einen Gewebeentnahmeteil des medizinischen Arbeitsinstruments öffnen und schließen.

In US 6,554,766 B2 ist eine Endoskopvorrichtung offenbart, die einen Einführteil mit einem krümmbaren Abschnitt, elektrische Antriebe zum Krümmen des krümmbaren Abschnitts, eine Operationseinheit zum Steuern der elektrischen Antriebe, eine Anschlussleitung zum Anschließen an eine externe Versorgungseinheit, ein Kupplungsteil und ein Halteteil zum Halten einer Halteeinheit an einer Operationsliege umfasst. Die Operationseinheit kann an dem Einführteil fixiert werden und kann vom Chirurgen zusammen mit diesem mit einer Hand gehalten werden. Die Operationseinheit weist einen konvexen Bedienbereich zur Steuerung des krümmbaren Abschnitts des Einfuhrteils auf, der vom Chirurgen mit dem Daumen der Hand bedient werden kann sowie Operationsschalter, die mit weiteren Fingern bedient werden können. Über die Halteeinheit und das Halteteil kann das Endoskop an der Operationsliege befestigt werden. Haltevorrichtungen zum Halten eines proximalen Endbereichs eines flexiblen Endoskops sind auch aus EP 1 859 724 A1 und aus US 6,569,084 B1 bekannt.

Zur Erleichterung des Einführens einer kolonoskopischen Vorrichtung wird gemäß US 2007/0135679 A1 ein Stabilisator in den Anus des Patienten eingesetzt. Der Stabilisator weist eine Öffnung auf, durch die die kolonoskopische Vorrichtung eingesetzt werden kann, sowie einen Hebel zum Blockieren der kolonoskopischen Vorrichtung relativ zum Stabilisator.

Die EP2332459A1 offenbart eine Endoskopievorrichtung für zwei Ärzte mit einem System zur Überwachung verschiedener Parameter, darunter der Griffkraft, die ein Arzt über ein Griffteil auf den Einführschaft ausübt. Der Schaft wird über einen an der Patientenliege befestigten Zugang in den Darm geführt und kann dabei auf Rollen laufen. Bei Überschreiten eines vorgegebenen Grenzwertes kann die Einführprozedur unterbrochen werden.

Es ist Aufgabe der vorliegenden Erfindung, ein endoskopisches System der eingangs genannten Art anzugeben, bei dem eine erleichterte Bedienung ermöglicht wird, insbesondere ein einfacheres und risikoärmeres Einführen des Schafts durch einen gekrümmten Zugangsweg in einen körperinneren Hohlraum bzw. in ein gekrümmtes Hohlorgan, sowie eine verbesserte Bedienung bei der Durchführung eines endoskopischen Eingriffs ermöglicht wird.

Diese Aufgabe wird durch ein endoskopisches System gemäß Anspruch 1 gelöst.

Vorteilhafte Weiterbildungen der Erfindung ergeben sich aus den Unteransprüchen.

Ein erfindungsgemäßes endoskopisches System umfasst ein flexibles Endoskop mit einem langerstreckten flexiblen Schaft und einem Kontrollteil. Der langerstreckte flexible Schaft ist zum Einführen in einen körperinneren Hohlraum, beispielsweise in ein Hohlorgan, durch eine natürliche oder eine künstlich geschaffene Körperöffnung ausgebildet. Der Schaft ist insbesondere zur Einführung in den Verdauungstrakt bei einer Koloskopie geeignet. Der Schaft, der im Folgenden auch als Einführschaft bezeichnet wird, weist an seinem distalen Ende eine Optik zur Erzeugung eines endoskopischen Bildes des körperinneren Hohlraums und in seinem Inneren Mittel zur Weiterleitung des endoskopischen Bildes vom distalen zum proximalen Endbereich auf. Das am proximalen Ende des Schafts angeordnete Kontrollteil kann Anschlüsse zur Versorgung des Endoskops sowie zur Weiterleitung des endoskopischen Bilds zu einer Bildverarbeitungs-, Anzeige- und/oder Speicherungseinrichtung aufweisen. Ferner kann das Kontrollteil Antriebe zur Abwinkelung des distalen Endbereichs des Einführschafts bzw. Kupplungen für derartige Antriebe aufweisen. Der Einführschaft kann weiterhin Spül- und Saugleitungen sowie Arbeitskanäle für endoskopische Arbeitsinstrumente aufweisen, für die entsprechende Anschlüsse bzw. Eingangsöffnungen am Kontrollteil angeordnet sein können.

Das endoskopische System umfasst ferner ein Bedienteil, das seitlich an den Schaft ansetzbar oder auf diesen aufschiebbar ist. Das Bedienteil ist somit insbesondere derart ausgebildet, dass es an den Schaft anlegbar oder über das distale Ende des Schafts auf diesen aufschiebbar ist und beispielsweise von einem Bediener mit einer Hand gemeinsam mit dem Schaft gehalten werden kann. Das Bedienteil weist mindestens ein Steuerelement auf.

Erfindungsgemäß umfasst das endoskopische System weiterhin einen an einer Patientenunterlage befestigbaren Schaftstopper, der einen durch das mindestens eine Steuerelement ansteuerbaren Arretierungsmechanismus zum Arretieren des Schafts gegen ein Verschieben in einer Längsrichtung des Schafts aufweist. Die Patientenunterlage kann beispielsweise eine Liege, ein Untersuchungs- oder ein Operationstisch sein, worauf der Patient bei einer mit dem endoskopischen System durchgeführten Untersuchung liegt. Als Patientenunterlage wird im vorliegenden Zusammenhang aber auch etwa ein Stuhl oder eine andere Einrichtung verstanden, die bei der durchgeführten Untersuchung oder Operation den Patienten trägt oder relativ zu der die Körperöffnung, durch die der flexible Schaft eingeführt wird, in einer festen räumlichen Beziehung steht. Der Schaftstopper kann beispielsweise auch über einen medizinischen Haltearm mit der Patientenunterlage verbunden sein. Der Schaftstopper ist derart ausgebildet, dass der Schaft des Endoskops in diesen eingeführt oder an diesen angelegt werden kann, so dass der Arretierungsmechanismus auf den Schaft einwirken kann. Der Arretierungsmechanismus kann beispielsweise als Klemmmechanismus ausgebildet sein, bei dem ein oder mehrere Klemmelemente, etwa eine Druckplatte und eine Gegendruckplatte, zwischen die der Schaft des Endoskops eingelegt werden kann, durch das Steuerelement ansteuerbar und zum Klemmen des Schafts motorisch verstellbar sind. Insbesondere sind alle Teile des Arretierungsmechanismus im Schaftstopper untergebracht. Vorteilhafterweise sind daher keine Einrichtungen am Endoskopschaft notwendig, um den Schaft mit dem Schaftstopper zu fixieren. Der Schaftstopper kann daher mit jedem konventionellen Endoskop verwendet werden.

Wird der Arretierungsmechanismus durch eine entsprechende Ansteuerung durch das Steuerelement aktiviert, so ist der Schaft gegen eine Verschiebung in Längsrichtung blockiert, wobei eine gewisse Beweglichkeit in einer Querrichtung des Schafts zulässig oder ebenfalls blockiert sein kann. In einem Betriebszustand des endoskopischen Systems wird somit der Arretierungsmechanismus durch das Steuerelement zum Arretieren oder Freigeben des Schafts bezüglich einer Bewegung in Längsrichtung gesteuert. Eine Drehung des Schafts um seine Längsachse ist im freigegebenen Zustand des Schafts möglich und kann im durch den Arretierungsmechanismus arretierten Zustand weiterhin möglich oder ebenfalls blockiert sein. In einem Montagezustand des endoskopischen Systems kann der Schaft zum Einführen in den Schaftstopper unabhängig vom Steuerelement stets in Längsrichtung verschiebbar sowie um seine Längsachse drehbar sein. Das Bedienteil ist an den Schaft proximalseitig des Schaftstoppers ansetzbar oder, bevor der Schaft in den Schaftstopper bzw. in den Arretierungsmechanismus eingeführt bzw. an diesen angesetzt worden ist, über das distale Ende des Schafts auf diesen aufschiebbar.

Dadurch, dass der Schaft durch den Arretierungsmechanismus des an der Patientenunterlage befestigbaren Schaftstoppers bezüglich seiner Längsrichtung fixiert werden kann, wird es ermöglicht, bei einem endoskopischen Eingriff eine einmal erreichte Einführposition des Schafts in ein körperinneres Hohlorgan zu fixieren. Der Bediener wird hierdurch von der Notwendigkeit, aktiv den Schaft in dieser Position zu halten, entlastet und kann unabhängig von dem Halten der Längsposition weitere Maßnahmen ergreifen, etwa eine Drehung des Schafts um seine Längsachse oder eine Abwinkelung des distalen Endes vornehmen, um eine nachfolgende Krümmung des Hohlorgans bei einem weiteren Einschieben des Schafts zu überwinden. Ebenso kann der Bediener, ohne den Schaft aktiv in der erreichten Längsposition halten zu müssen, chirurgische Manipulationen mit Hilfe von durch den Schaft geführten Arbeitsinstrumenten vornehmen. Weiterhin wird dadurch, dass das Bedienteil an den Schaft ansetzbar oder auf diesen aufschiebbar ist, das Vorschieben und Zurückziehen bzw. Drehen des Schafts erleichtert, insbesondere dann, wenn das Bedienteil dann, wenn es seitlich an den Schaft angesetzt oder auf diesen aufgeschoben ist, gemeinsam mit dem Schaft mit einer Hand umgriffen und gemeinsam mit diesem in Längsrichtung vor- und zurückbewegt bzw. um die Längsachse des Schafts gedreht werden kann. Schließlich wird dadurch, dass das Bedienteil das Steuerelement zum Arretieren bzw. Freigeben des Schafts hinsichtlich einer Verschiebung in Längsrichtung aufweist, die Bedienung des endoskopischen Systems weiter erleichtert, da das Bedienteil mit dem Steuerelement gemeinsam mit dem Schaft in Längsrichtung verschoben werden kann. Auf diese Weise ist eine erhebliche Erleichterung für den Chirurgen und eine damit verbundene Risikoverminderung erreichbar.

Gemäß einer bevorzugten Ausführungsform der Erfindung weist das Bedienteil eine halbschalenförmige Ausnehmung zur Aufnahme des Schafts auf. Wenn das Bedienteil an den Schaft angesetzt ist, ist der Schaft somit in der Halbschale aufgenommen, so dass das Bedienteil den Schaft zumindest teilweise seitlich umschließt. Hierdurch wird erreicht, dass das Bedienteil seitlich an den Schaft ansetzbar ist und der Schaft sicher am Bedienteil gehalten werden kann und dabei zusätzlich eine Führung in Längsrichtung erfährt. In besonders bevorzugter Weise ist das Bedienteil mit der Halbschale derart ausgebildet, dass das Bedienteil zusammen mit dem in die Halbschale eingesetzten Schaft mit einer Hand gegriffen und gleichzeitig der Schaft fest in die Halbschale gepresst werden kann, so dass ein ausreichender Reibschluss zwischen der Hand des Bedieners, dem Schaft und dem Bedienteil besteht, um den Schaft in seiner Längsrichtung zu verschieben und/oder um seine Längsachse zu drehen. Hierdurch wird auf besonders einfache Weise die Handhabung des Schafts durch den Bediener mit einer einzigen Hand ermöglicht.

Zusätzlich oder alternativ zur halbschalenförmigen Aufnahme kann ein Umgriffelement vorgesehen sein, mit dem das Bedienteil fest, insbesondere in Längsrichtung ausreichend fest, zum Bewerkstelligen einer Längsverschiebung des Schafts bzw. einer Drehung des Schafts um seine Längsachse, an dem Schaft befestigbar ist. Das Bedienteil kann in diesem Fall den Schaft umschließen. Auch hierdurch wird eine besonders einfache und sichere Handhabung des Einführschafts mit einer einzigen Hand ermöglicht.

Gemäß der Erfindung ist, sofern das Bedienteil nur ein einziges Steuerelement aufweist, dieses als Schaftsensor zum Erfassen des Anliegens des Schafts am Bedienteil ausgebildet; sofern das Bedienteil mehrere Steuerelemente aufweist, ist mindestens eines der Steuerelemente derart ausgebildet. Dabei ist der Arretierungsmechanismus derart durch den Schaftsensor steuerbar, dass dann, wenn das Bedienteil nicht an den Schaft angesetzt oder auf diesen aufgeschoben ist, der Schaft arretiert ist. Dies bedeutet, dass in einem Betriebszustand des endoskopischen Systems der Schaft nur dann hinsichtlich einer Verschiebung in Längsrichtung relativ zum Schaftstopper freigegeben werden kann, wenn das Bedienteil an den Schaft angesetzt oder auf diesen aufgeschoben ist. Eine mögliche Freigabe des Schafts gegenüber dem Schaftstopper erfolgt somit aufgrund eines Signals des Schaftsensors, das anzeigt, ob der Schaft an dem Bedienteil anliegt. Hierdurch kann sichergestellt werden, dass in einem Betriebszustand des Systems eine Längsverschiebung des Schafts immer dann ausgeschlossen ist, wenn das Bedienteil nicht mit dem Schaft verbunden ist, in welchem Fall darauf geschlossen werden kann, dass ein Bediener den Schaft nicht zum Halten oder Verschieben ergriffen hat.

Vorzugsweise ist der Schaftsensor als Drucksensor ausgebildet. Insbesondere ist der Drucksensor an einer inneren Oberfläche der Halbschale angeordnet. Hierdurch kann nicht nur besonders sicher erfasst werden, ob der Schaft an dem Bedienteil anliegt, sondern auch, ob das Bedienteil und der Schaft von einem Bediener fest aneinander angedrückt werden. Dadurch kann erreicht werden, dass der Schaft zur Verschiebung in Längsrichtung relativ zum Schaftstopper nur dann freigegeben ist oder gemäß weiterer Bedienungseingaben freigegeben werden kann, wenn der Schaft und das Bedienteil von einem Bediener mit einer für eine Kontrolle der Längsverschiebung des Schafts ausreichenden Kraft ergriffen worden sind. Insbesondere kann hierdurch vermieden werden, dass eine Freigabe des Schafts bezüglich der Längsverschiebung zu einem Zeitpunkt erfolgt, zu dem der Bediener keine ausreichende Kontrolle über den Schaft hat.

Gemäß einer weiteren bevorzugten Ausführungsform der Erfindung ist in dem Fall, dass das Bedienteil nur ein einziges Steuerelement aufweist, dieses als Bedienelement zum manuellen Steuern des Arretierungsmechanismus ausgebildet; in dem Fall, dass das Bedienteil mehrere Steuerelemente aufweist, ist mindestens eines der Steuerelemente derart ausgebildet, wobei beispielsweise ein anderes Steuerelement als Schaftsensor ausgebildet sein kann. Ein Bedienelement zum manuellen Steuern des Arretierungsmechanismus kann beispielsweise als Druckknopf oder als Bedienhebel (Joystick) ausgebildet sein und am Bedienteil in vorteilhafter Weise derart angeordnet sein, dass es von einem Bediener, der das Bedienteil gemeinsam mit dem Schaft mit einer Hand umgriffen hält, mit einem Finger derselben Hand betätigt werden kann. Hierdurch kann in einfacher Weise eine manuelle Bedienung des Arretierungsmechanismus zur Erleichterung des Einsatzes des endoskopischen Systems ermöglicht werden.

In vorteilhafter Weise weist das Bedienteil mindestens zwei Steuerelemente auf, nämlich sowohl mindestens einen Schaftsensor, der beispielsweise als in einer zur Aufnahme des Schafts vorgesehenen Halbschale angeordneter Drucksensor ausgebildet sein kann, als auch mindestens ein Bedienelement zum manuellen Steuern des Arretierungsmechanismus. Der mindestens eine Schaftsensor und das mindestens eine Bedienelement sind hierbei derart geschaltet, dass nur dann, wenn das Signal des Schaftsensors das Vorhandensein des Schafts am Bedienteil anzeigt, der Arretierungsmechanismus durch das Bedienelement steuerbar ist. Dies bedeutet, dass im Betriebszustand des endoskopischen Systems immer dann, wenn das Signal des Schaftsensors anzeigt, dass das Bedienteil nicht an den Schaft angesetzt bzw. nicht auf diesen aufgeschoben ist, der Arretierungsmechanismus den Schaft hinsichtlich einer Längsverschiebung blockiert, und zwar unabhängig von einer Betätigung des mindestens einen Bedienelements. Andererseits ist im Betriebszustand dann, wenn gemäß dem Signal des Schaftsensors das Bedienteil an den Schaft angesetzt oder auf diesen aufgeschoben ist, das mindestens eine Bedienelement aktiv, d.h. der Arretierungsmechanismus ist durch dieses ansteuerbar und der Schaft kann wahlweise arretiert oder freigegeben werden. In einem Montagezustand des endoskopischen Systems kann ein Verschieben des Schafts in seiner Längsrichtung unabhängig vom Anliegen des Schafts am Bedienteil möglich sein. Dadurch, dass das Bedienteil sowohl einen Schaftsensor als auch ein Bedienelement zum Ansteuern des Arretierungsmechanismus aufweist und dass das Bedienelement nur dann ein Steuern des Arretierungsmechanismus gestattet, wenn ein Signal des Schaftsensors das Vorhandensein des Schafts am Bedienteil anzeigt, kann eine besonders sichere und komfortable Bedienung des endoskopischen Systems ermöglicht werden. Insbesondere erfolgt eine automatische Fixierung des Schafts im oder am Schaftstopper, wenn das Bedienteil nicht an den Schaft angesetzt bzw. nicht auf diesen aufgeschoben ist und somit darauf geschlossen werden kann, dass der Bediener nicht zum Ausführen einer Längsverschiebung des Schafts bereit ist. Anderseits wird dann, wenn der Schaft am Bedienteil anliegt und wenn insbesondere aus dem Signal eines als Drucksensor ausgebildeten Schaftsensors geschlossen werden kann, dass der Bediener das Bedienteil und den Schaft mit ausreichender Kraft umgriffen hält, eine vollständige manuelle Kontrolle der Längsverschiebung des Schafts ermöglicht.

Vorzugsweise umfasst das Bedienteil ein oder mehrere Bedienelemente zum Steuern weiterer Funktionen des endoskopischen Systems. Gemäß einer besonders bevorzugten Ausführungsform der Erfindung weist das Bedienteil ein Bedienelement, etwa einen Kippschalter,einen Joystick oder einen Touchscreen, zur Steuerung der Abwinkelung des distalen Endabschnitts des Schafts auf. Das Bedienteil kann auch beispielsweise als Bedienelement eine Menüsteuerung aufweisen, die mehrere oder sogar alle Funktionen des Endoskops mit einer Hand zu bedienen gestattet. Vorzugsweise ist das Bedienelement bzw. sind die Bedienelemente derart angeordnet, dass eine Betätigung durch einen Bediener, der das Bedienteil und den Schaft mit einer Hand hält, mit derselben Hand möglich ist. Hierdurch wird die Bedienung des endoskopischen Systems weiter vereinfacht.

In vorteilhafter Weise kann ein weiteres Bedienteil vorgesehen sein, das insbesondere Bedienelemente zur Steuerung eines oder mehrerer endoskopischer Arbeitsinstrumente aufweisen kann, die durch den Schaft des Endoskops zum körperinneren Hohlraum geführt werden können. Die zu steuernden Funktionen der endoskopischen Arbeitsinstrumente umfassen insbesondere eine Längsverschiebung in Richtung des Einführschafts sowie die Betätigung eines am distalen Ende eines endoskopischen Arbeitsinstruments angeordneten Werkzeugs, etwa einer Zange, einer Schere oder einer Schlinge, womit in dem körperinneren Hohlraum Manipulationen etwa zur Entnahme einer Gewebeprobe oder zur Durchführung einer chirurgischen endoskopischen Operation vorgenommen werden können. Hierfür können entsprechend ausgebildete motorische Antriebe der endoskopischen Arbeitsinstrumente vorgesehen sein. Dadurch, dass ein weiteres Bedienteil vorhanden ist, das vom Bediener mit seiner zweiten Hand umgriffen werden kann, wird eine einfache und vollständige Bedienung des endoskopischen Systems durch einen einzigen Bediener ermöglicht. Das weitere Bedienteil kann auch als Menüsteuerung ausgebildet sein, die wahlweise auf das zuerst genannte Bedienteil aufgesetzt werden kann oder von diesem getrennt bedienbar ist.

Alternativ kann in vorteilhafter Weise auch ein einziges Bedienteil vorgesehen sein, das sowohl wie oben beschrieben zur Steuerung der Funktionen des endoskopischen Systems als auch zur Steuerung der genannten Funktionen eines oder mehrerer endoskopischer Arbeitsinstrumente ausgebildet ist.

Zur Ansteuerung des Arretierungsmechanismus kann das Steuerelement durch eine elektrische Leitung direkt oder, über eine Steuerungseinrichtung des endoskopischen Systems, indirekt mit dem Arretierungsmechanismus verbunden sein. In vorteilhafter Weise ist der Arretierungsmechanismus über eine drahtlose Verbindung durch das Steuerelement ansteuerbar. Eine solche drahtlose Verbindung kann beispielsweise durch eine drahtlose Verbindung zwischen dem Bedienteil und einer Steuerungseinrichtung und eine drahtlose oder drahtgebundene Verbindung zwischen der Steuerungseinrichtung und dem Arretierungsmechanismus realisiert sein oder auch durch eine drahtgebundene Verbindung zwischen dem Bedienteil und der Steuerungseinrichtung und eine drahtlose Verbindung zwischen der Steuerungseinrichtung und dem Arretierungsmechanismus. Auch ein weiteres Bedienteil kann drahtlos mit der Steuerungseinrichtung verbunden sein. Hierdurch werden ein einfacher und sicherer Aufbau sowie eine besonders komfortable Bedienung des endoskopischen Systems ermöglicht.

Gemäß einer bevorzugten Ausführungsform der Erfindung weist der Schaftstopper eine Durchgangsöffnung auf, die zum Halten des Schafts in einer Querrichtung des Schafts ausgebildet ist. Der Schaft wird beim Aufbau des Systems durch die Durchgangsöffnung hindurchgeführt, oder der Schaft wird in eine Ausnehmung des Schaftstoppers eingelegt und in dieser durch einen Verschluss, etwa durch einen Bügel oder ein elastisches Element, gehalten, wobei die Ausnehmung zusammen mit dem Verschluss die Durchgangsöffnung bildet. Die Durchgangsöffnung kann ausgebildet sein, um unterschiedliche Richtungen des Schafts zuzulassen, oder es kann der Schaftstopper eine Führung aufweisen, durch die der Schaft in einer vorgegebenen Richtung geführt wird. Die Durchgangsöffnung kann auch beispielsweise durch eine in einem begrenzten Winkelbereich schwenkbare Haltebuchse, durch die der Schaft führbar ist, gebildet werden. Eine Drehung des Schafts um seine Längsachse ist dabei vorzugsweise weiterhin möglich. Hierdurch wird eine weitere Verbesserung der Handhabung des endoskopischen Systems ermöglicht, insbesondere wird es ermöglicht, dass der Einführschaft in einem Bereich zwischen dem Schaftstopper und der Körperöffnung, durch die der Schaft eingeführt wird, derart gehalten bzw. geführt ist, dass auch bei einer Schubbewegung zum Vorschieben des Schafts ein Ausweichen oder Buckeln des Schafts sicher vermieden werden kann.

Weiterhin ist es bevorzugt, dass der Schaftstopper distalseitig des Arretierungsmechanismus bzw. distalseitig der Durchgangsöffnung einen Schlauchhalter zum Halten einer schlauchförmigen Schaftschutzfolie aufweist, wobei auch beispielsweise eine Haltebuchse, durch die der Schaft führbar ist, als Schlauchhalter ausgebildet sein kann. Die Schaftschutzfolie kann derart über den Einführschaft gezogen werden, dass sie mit einem proximalen Ende im proximalen Endbereich des Einführschafts an diesem befestigt werden kann und ein distales Ende der Schaftschutzfolie vom Schlauchhalter getragen wird. Der Bereich zwischen dem Kontrollteil und dem Schaftstopper ist somit von der Schaftschutzfolie umgeben; das Bedienteil ist an den von der Schaftschutzfolie umgebenen Schaft ansetzbar. Beim Vorschieben bzw. Zurückziehen des Schafts ist stets derjenige Abschnitt des Einführschafts, der sich proximalseitig des Schaftstoppers befindet, von der Schaftschutzfolie umgeben; ebenso ist der innerhalb des Schaftstoppers befindliche Teil des Schafts von dieser umgeben. Eine etwaige Kontamination des Einführschafts kann sich beim Herausziehen des Einführschafts aus dem körperinneren Hohlraum somit nicht auf den Schaftstopper und das Bedienteil auswirken und führt auch nicht zu einer Kontamination der betreffenden Bereiche der Patientenunterlage. Der Schaftstopper mit dem Schlauchhalter kann nahe genug an der Körperöffnung, durch die der Schaft eingeführt wird, angeordnet werden, so dass der zwischen der Körperöffnung und dem Schlauchhalter des Schaftstoppers verlaufende, freiliegende Teilbereich des Schafts nicht in Berührung mit der Patientenunterlage gelangt, so dass auch diesbezüglich eine Kontamination vermieden werden kann. Ist der Einführschaft vollständig aus der Körperöffnung herausgezogen, so kann die Schaftschutzfolie, die hierzu zweckmäßig mit einer Überlänge gegenüber der Länge des Einführschafts ausgestattet ist, über dessen distales Ende gezogen werden, so dass der Einführschaft vollständig eingehüllt ist und zur Reinigung und Dekontamination transportiert werden kann.

Gemäß einer besonders bevorzugten Ausführungsform der Erfindung umfasst das Kontrollteil Versorgungsanschlüsse und/oder Antriebskupplungen des Endoskops und ist an eine Versorgungseinrichtung, die ebenfalls Teil des endoskopischen Systems sein kann, ankoppelbar. Die Versorgungsanschlüsse sind insbesondere Anschlüsse zur Übertragung des Beleuchtungslichts und zur elektrischen Energieversorgung des Endoskops sowie Anschlüsse zur Übertragung eines Videosignals an eine Bildverarbeitungs-, Anzeige- und Speicherungseinrichtung und Anschlüsse für die Einleitung einer Spülflüssigkeit in einen Spülkanal des Endoskops und die Absaugung aus einem Saugkanal des Endoskops. Das Kontrollteil kann Antriebskupplungen aufweisen zur Verbindung von Antrieben bzw. Übertragungsmitteln zur Abwinkelung des distalen Endbereichs des Einführschafts, etwa Seilzügen, mit motorischen Antrieben, die der Versorgungseinrichtung zugeordnet sind. Die Versorgungseinrichtung kann weiterhin motorische Antriebe für endoskopische Arbeitsinstrumente aufweisen, die ebenfalls an entsprechende Kupplungen der Arbeitsinstrumente bzw. des Kontrollteils anschließbar sind. Insbesondere kann das Kontrollteil ein geschlossenes Gehäuse aufweisen, das auf seiner Außenseite die Versorgungsanschlüsse bzw. Antriebskupplungen trägt. Hierdurch wird eine Versorgung des Endoskops sowie eine einfache Ausgestaltung, wobei das Endoskop selbst keine motorischen Antriebe aufweisen muss, ermöglicht.

Vorzugsweise ist der Schaft drehbar mit dem Kontrollteil bzw. mit dem Gehäuse des Kontrollteils verbunden. Hierdurch wird es ermöglicht, den Einführschaft in einer zur Einführung durch einen gekrümmten Zugangsweg bzw. in ein gekrümmtes Hohlorgan geeigneten Richtung zu drehen, um jeweils eine optimale Abwinkelung des distalen Endabschnitts einstellen zu können. Ebenso wird hierdurch die Wahl einer jeweils optimalen Ausrichtung der Optik bzw. der Werkzeuge der endoskopischen Arbeitsinstrumente ermöglicht. Zur Erleichterung der Rotation des Schafts um seine Längsachse kann angrenzend an das Kontrollteil ein Drehgriff oder ein seitlich vom Schaft abstehender Drehhebel angeordnet sein. Vorzugsweise ist die Drehung des Schafts relativ zum Kontrollteil auf einen begrenzten Winkelbereich eingeschränkt, beispielsweise auf maximal ±180°, um eine übermäßige Verdrillung der innerhalb des Schafts und des Kontrollteils geführten Leitungen zu vermeiden. Ferner kann der Schaft lösbar mit dem Kontrollteil bzw. dem Gehäuse des Kontrollteils verbunden sein, um die Reinigung und/oder Sterilisation des Schafts zu vereinfachen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 ein Ausführungsbeispiel eines erfindungsgemäßen endoskopischen Systems im Überblick;
Fig. 2a und 2b zwei teilweise geschnittene Seitenansichten des Schaftstoppers und des Bedienteils gemäß einem Ausführungsbeispiel der Erfindung in zwei Betriebszuständen;
Fig. 3a und 3b eine alternative Ausführungsform des Schaftstoppers in einer teilweise geschnittenen Seitenansicht (Fig. 3a) und einer Ansicht von oben (Fig. 3b);
Fig. 4a und 4b das Bedienteil aus Fig. 2a und 2b in einer perspektivischen Ansicht von unten sowie beim Betrieb mit einer Hand eines Bedieners;
Fig. 5a und 5b ein Kontrollteil gemäß einem weiteren Ausführungsbeispiel des endoskopischen Systems in zwei perspektivischen Ansichten;
Fig. 6 das Kontrollteil aus Fig. 5a und 5b mit einem Versorgungsadapter einer Versorgungseinrichtung.

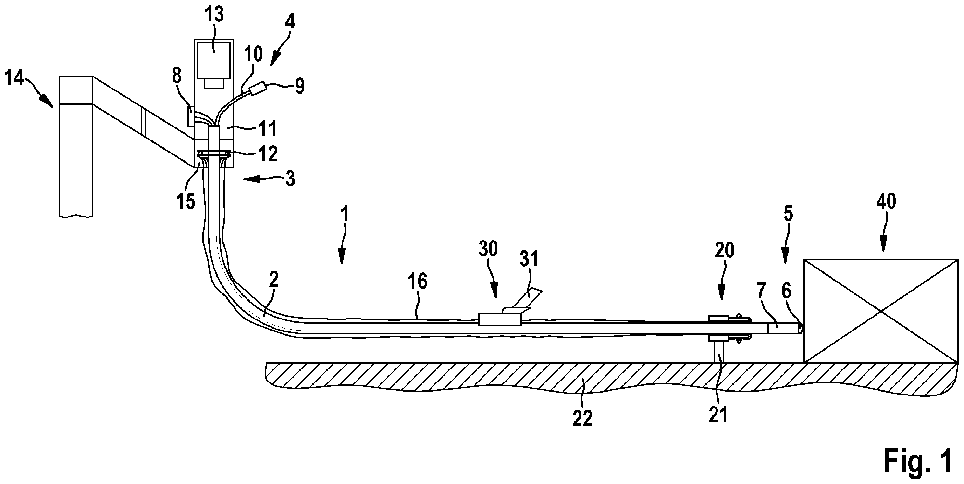

Wie in Fig. 1 schematisch im Überblick gezeigt ist, umfasst ein erfindungsgemäßes endoskopisches System gemäß einem Ausführungsbeispiel ein flexibles Endoskop 1 mit einem langerstreckten flexiblen Schaft 2 und einem am proximalen Ende 3 des Schafts 2 anschließenden Kontrollteil, das als Endoskopbox 4 ausgebildet ist. Am distalen Ende 5 des Einführschafts 2 ist ein Endoskopobjektiv 6 zur Erzeugung eines endoskopischen Bilds angeordnet, das beispielsweise durch einen elektronischen Bildaufnehmer aufgenommen und über innerhalb des Schafts 2 verlaufende elektrische Leitungen zur Endoskopbox 4 übermittelt wird. Der distale Endbereich 7 des Schafts 2 ist steuerbar ausgebildet und kann über innerhalb des Schafts 2 verlaufende Seilzüge in einer oder mehreren Richtungen quer zur Längsachse des distalen Endabschnitts 7 abgewinkelt werden.

Ferner enthält der Schaft 2 Lichtleiter zur Weiterleitung von an der Endoskopbox 4 eingekoppeltem Beleuchtungslicht zum distalen Ende 5 sowie Saug- und Spülkanäle, um Luft bzw. Flüssigkeit zum distalen Ende 5 zu führen und von dort wieder abzusaugen, und einen Arbeitskanal zur Durchführung eines endoskopischen Arbeitsinstruments durch den Schaft 2 bis zu dessen distalem Ende 5. Die Endoskopbox 4 weist einen Anschluss 8 für ein Videokabel zum Anschluss an eine externe Videoeinheit auf sowie in Fig. 1 nicht gezeigte Anschlüsse für ein Lichtkabel zum Anschluss einer externen Lichtquelle und Spül- und Sauganschlüsse. Im Bereich der Endoskopbox 4 befindet sich ferner eine Öffnung 9 des Arbeitskanals an einer abgewinkelten Arbeitskanalführung 10, in die das endoskopische Arbeitsinstrument eingeschoben werden kann. Der Videoanschluss und die weiteren nicht dargestellten Anschlüsse für das Beleuchtungslicht und die Saug- und Spülleitungen sind auf der Außenseite eines Gehäuses 11 angeordnet, an dem über ein Drehlager 12 der Schaft 2 drehbar befestigt ist. Um ein übermäßiges Verdrillen der durch das Drehlager 12 verlaufenden Kabel, Leitungen und Kanäle zu vermeiden, ist die Rotation des Schafts 2 um seine Längsachse am Drehlager 12 durch in Fig. 1 nicht dargestellte Anschläge auf einen Winkelbereich von ca. ±180° begrenzt. Zur Steuerung der Abwinkelung des distalen Endabschnitts 7 des Schafts 2 kann ebenfalls innerhalb des Gehäuses 11 der Endoskopbox 4 ein in Fig. 1 symbolisch dargestellter motorischer Antrieb 13 aufgenommen sein. Gemäß einer in Fig. 1 nicht dargestellten Variante können aber auch Kupplungen zur Übertragung einer Antriebsbewegung von einem externen motorischen Antrieb vorgesehen sein. Das Gehäuse 11 kann weitere Komponenten, etwa einen Prozessor und die Beleuchtungslichtquelle, enthalten. Gemäß der in Fig. 1 gezeigten Variante wird die Endoskopbox 4 durch einen Haltearm 14 gehalten, der an einem Verbindungsabschnitt 15 der Endoskopbox 4 angreift, der auch das Drehlager 12 zum Halten des proximalen Endes 3 des Schafts 24 aufweist. Die Gesamtlänge des Schafts 2 beträgt beispielsweise bei einem für eine Koloskopie geeigneten System ca. 150 cm oder mehr.

Das endoskopische System umfasst ferner einen Schaftstopper 20, der in Fig. 1 nur symbolisch dargestellt ist und der über eine federnde Halterung 21 an einer Patientenliege 22 befestigt werden kann. Ferner umfasst das endoskopische System ein von einem Bediener mit einer Hand haltbares Bedienteil 30, das auf den Schaft 2 aufgesetzt werden kann und auf das, wie in Fig. 1 dargestellt, eine Menüsteuerung 31 aufgesetzt werden kann, die weitere Bedienelemente aufweist. Der Schaftstopper 20 und das Bedienteil 30 sind unten näher erläutert.

Wie in Fig. 1 gezeigt, wird der Schaftstopper 20 zur Durchführung einer endoskopischen Untersuchung oder Operation in einem geeigneten Abstand von beispielsweise ca. 15 - 20 cm von einem zu untersuchenden bzw. zu operierenden Patienten 40, der auf der Patientenliege 22 gelagert ist, an dieser befestigt. Dabei ist die Körperöffnung, durch die der Schaft 2 des Endoskops 1 zur Durchführung des vorgesehenen Eingriffs in einen körperinneren Hohlraum des Patienten einzuführen ist, zum Schaftstopper 20 gerichtet, und die Halterung 21 derart eingerichtet, dass der durch den Schaftstopper 20 geführte Schaft 2 im Wesentlichen gerade in den körperinneren Hohlraum eingeführt werden kann. Das in Fig. 1 gezeigte endoskopische System ist insbesondere zur Durchführung eines kolonoskopischen Eingriffs eingerichtet.

Der Schaft 2 wird in den Schaftstopper 20 eingelegt oder mit seinem distalen Endabschnitt 7 durch den Schaftstopper 20 hindurchgeführt. Proximalseitig des Schaftstoppers 20 wird das Bedienteil 30 auf den Schaft 2 aufgesetzt, wobei ein Abstand zwischen dem Schaftstopper 20 und dem Bedienteil 30 verbleibt. Der Abstand ist so gewählt, dass gemeinsam mit dem Bedienteil 30 ein Vorschub des Schafts 2 möglich ist, ohne dass ein Ausweichen oder Buckeln des Schafts zwischen dem Bedienteil 30 und dem Schaftstopper 20 auftritt. Das Bedienteil 30 weist einen unten näher erläuterten Schaftsensor auf, der mit einem Arretierungsmechanismus des Schaftstoppers 20 derart gekoppelt ist, dass der Schaft 2 innerhalb des Schaftstoppers 20 nur dann in Längsrichtung, d.h. zum Einführen in den bzw. zum Herausziehen aus dem körperinneren Hohlraum des Patienten 40, verschiebbar ist, wenn das Bedienteil 30 auf den Schaft 2 aufgesetzt ist und fest an diesen angedrückt wird. Die Endoskopbox 4 ist derart gehalten, dass der Schaft 2 des Endoskops 1 in den körperinneren Hohlraum eingeschoben werden kann, wofür etwa eine eine genügende Länge des Schafts umfassende Krümmung im Bereich zwischen dem Bedienteil 30 und der Endoskopbox 4 vorgesehen sein kann, oder auch beispielweise die Endoskopbox 4 mit dem Haltearm 14 näher an den Patienten heran bewegbar sein kann. In diesem Zustand kann der Schaft 2 in Längsrichtung zum Patienten 40 oder vom Patienten 40 weggerichtet verschoben werden, andernfalls ist der Schaft 2 im Schaftstopper 20 blockiert.

Der Schaft 2 des Endoskops 1 ist von einer schlauchförmigen Schaftschutzfolie 16 umgeben, die proximalseitig am proximalen Ende 3 des Schafts 2 befestigt ist, beispielsweise durch Kleben bzw. mit Hilfe eines Klebebands. Distalseitig ist die Schaftschutzfolie 16 am Schaftstopper 20 gehalten und kann hierfür eine entsprechende Verstärkung aufweisen. Die Schaftschutzfolie 16 weist auf ihrer Innenseite sowie ggf. auf ihrer Außenseite einen Gleitbelag auf, um das Verschieben des von ihr umschlossenen Schafts 2 innerhalb des Schaftstoppers 20 erleichtern. Dabei bewegt sich die Schaftschutzfolie 16 bei einer Verschiebung des Schafts 2 nicht oder nur wenig innerhalb des Schaftstoppers 20, vielmehr wird die Schaftschutzfolie 16 proximalseitig des Schaftstoppers 20 beim Vorschieben des Einführschafts 2 gerafft und beim Zurückziehen des Einführschafts 2 geglättet.

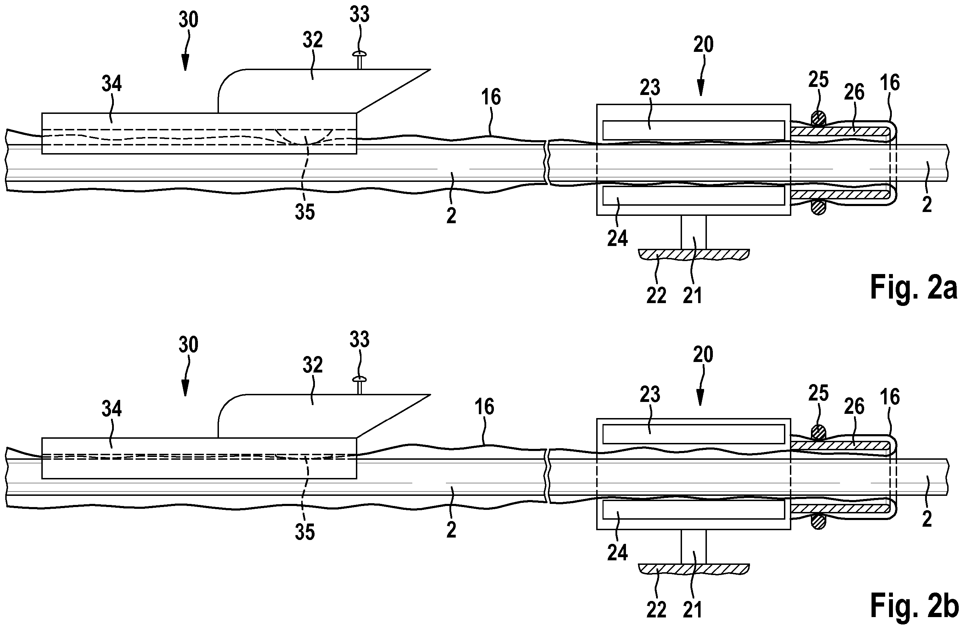

Wie in Fig. 2a gezeigt, ist bei einer ersten Ausführungsform des Schaftstoppers 20 ein erster Abschnitt des Schafts 2 durch den Schaftstopper 20 geführt, der mit der Halterung 21 an der Patientenliege 22 befestigt ist. Der Schaftstopper 20 umfasst einen Arretierungsmechanismus, der durch eine bewegliche Druckplatte 23 und eine feststehende Gegendruckplatte 24 gebildet wird, wobei die bewegliche Druckplatte 23 motorisch in Richtung auf die Gegendruckplatte 24 verstellbar und von dieser wegbewegbar ist. Die Druckplatte 23 und die Gegendruckplatte 24 können mit einer reibungserhöhenden Oberfläche sowie einer Polsterung zur Erhöhung der Reibung mit dem zwischen diesen eingelegten Schaftabschnitt und zur Vermeidung von Beschädigungen des Schafts versehen sein. Zur Erleichterung des Einlegens des Schafts 20 zwischen die Druckplatte 23 und die Gegendruckplatte 24 kann beispielsweise die Druckplatte 23 an einem schwenkbaren Halter befestigt sein (nicht dargestellt). Der Schaftstopper 20 weist ferner einen flexiblen Bügel 25 auf, der eine Durchgangsöffnung bildet, durch die der Schaft 2 geführt wird. Distalseitig ist am Schaftstopper 20 ein Schlauchhalter 26 angeordnet, über den die Schaftschutzfolie 16 gezogen ist. Die Schaftschutzfolie 16 wird am Schaftstopper 20 ebenfalls durch den Bügel 25 gehalten, wobei eine in Fig. 2a und 2b nicht gezeigte Längenreserve der Schaftschutzfolie vorgesehen sein kann.

Zum Arretieren des Schafts 2 bezüglich einer Verschiebung in seiner Längsrichtung wird die bewegliche Druckplatte 23 motorisch in Richtung auf die Gegendruckplatte 24 bewegt und der Schaft 2 somit zwischen der Druckplatte 23 und der Gegendruckplatte 24 geklemmt und dadurch blockiert. Eine gewisse seitliche Bewegung sowie ein Verschwenken des Schafts kann durch eine federnde Ausbildung der Halterung 21 weiterhin ermöglicht werden.

Das Bedienteil 30 gemäß Fig. 2a und 2b umfasst einen Träger 32, der einen Joystick 33 trägt. Auf der Unterseite des Trägers 32 ist eine Halbschale 34 angebracht, die zur Aufnahme des Schafts 2 geformt ist. An der Innenseite der Halbschale 34 befindet sich ein Drucksensor 35, der durch ein festes Andrücken des Schafts 2 betätigt werden kann. Das Bedienteil kann vom Bediener gemeinsam mit dem in die Halbschale 34 eingelegten Abschnitt des Schafts 2 mit einer Hand umgriffen und der Schaft 2 dabei in die Halbschale 34 hineingedrückt werden, so dass der Drucksensor 35 betätigt wird. Gleichzeitig mit dem Halten des Schafts 2 am Bedienteil 30 kann der Bediener mit einem Finger den Joystick 33 betätigen.

In der in Fig. 2a gezeigten Situation ist der Schaft 2 nicht fest am Bedienteil 30 angelegt. Durch den Drucksensor 35 wird somit kein Signal abgegeben, das das Vorhandensein des Schafts 2 anzeigt. Der Drucksensor 35 ist über eine drahtlose Verbindung mit einer nicht dargestellten Steuerungseinrichtung verbunden, die den Arretierungsmechanismus des Schaftstoppers 20 steuert. Die Steuerungseinrichtung ist derart eingerichtet, dass dann, wenn vom Drucksensor kein Signal empfangen wird, das das Vorhandensein des Schafts 2 anzeigt, der Arretierungsmechanismus stets geschlossen ist, d.h. die Druckplatte 23 zum Klemmen des Schafts 2 gegen die Gegendruckplatte 24 angesteuert wird.

In der in Fig. 2b dargestellten Situation ist der Schaft 2 in die Halbschale 34 eingelegt und wird fest an diese angedrückt, so dass der Drucksensor 35 ein Signal erzeugt, das das Vorhandensein des Schafts 2 anzeigt. In diesem Fall wird durch die Steuerungseinrichtung die Druckplatte 23 in die Gegenrichtung bewegt und dadurch vom Schaft 2 gelöst, so dass dieser gemeinsam mit der Schaftschutzfolie 16 frei in Längsrichtung verschiebbar ist.

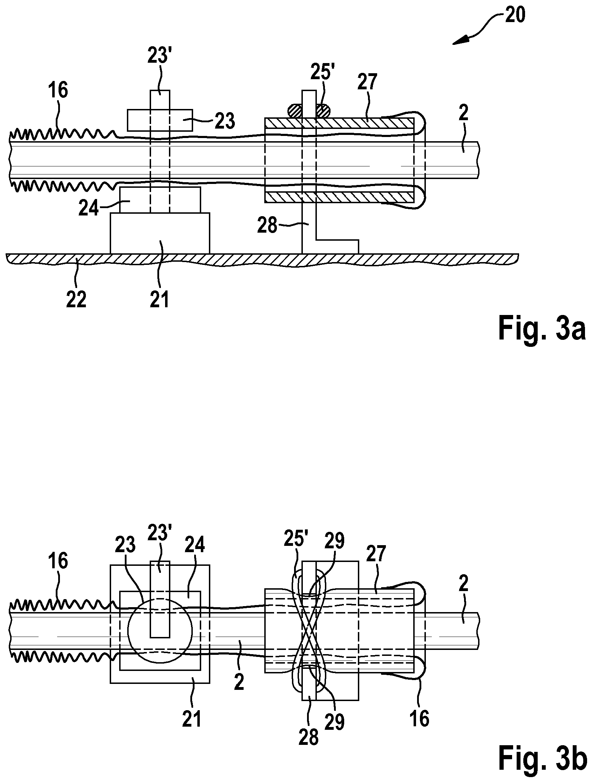

Bei der in Fig. 3a und 3b in zwei Ansichten dargestellten zweiten Ausführungsform des Schaftstoppers umfasst dieser einen Arretierungsmechanismus, der mit der Halterung 21 an der Patientenliege 22 befestigt ist, und eine von dem Arretierungsmechanismus getrennte, beispielsweise gegenüber diesem auf einer Schiene verschiebbare Schaftführung. Der Arretierungsmechanismus wird durch eine bewegliche Druckplatte 23 und eine feststehende Gegendruckplatte 24 gebildet, wobei die bewegliche Druckplatte 23 motorisch in Richtung auf die Gegendruckplatte 24 verstellbar und von dieser wegbewegbar ist, um den Schaft 2 gegenüber einer Verschiebung in Längsrichtung zu arretieren bzw. freizugeben. Die Druckplatte 23 ist an einem Schwenkarm 23' befestigt. Die Schaftführung umfasst eine Haltebuchse 27, durch die der Schaft 2 geführt wird. Die Haltebuchse 27 ist mit einem Gummiband 25' in einer Gabel 28 gehalten, die an der Patientenliege 22 befestigt ist. Einander gegenüberliegende Kerben 29 in der Außenseite der Haltebuchse 27 ermöglichen ein spielbehaftetes Halten der Haltebuchse 27 in der Gabel 28, so dass ein Schwenken der Haltebuchse 27 in einem begrenzten Winkelbereich und damit ein Verstellen der Führung des Schafts 2 in eine gewünschte Richtung ermöglicht wird. Distalseitig ist die Schaftschutzfolie 16 auf der Außenseite der Haltebuchse 27 befestigt, beispielsweise festgeklebt. Proximalseitig weist die Schaftschutzfolie 16 eine Längenreserve auf, die durch die Raffung der Schaftschutzfolie 16 in Fig. 3a und 3b angedeutet ist. Im Übrigen ist das endoskopische System wie in Fig. 1 bis 2b dargestellt ausgebildet.

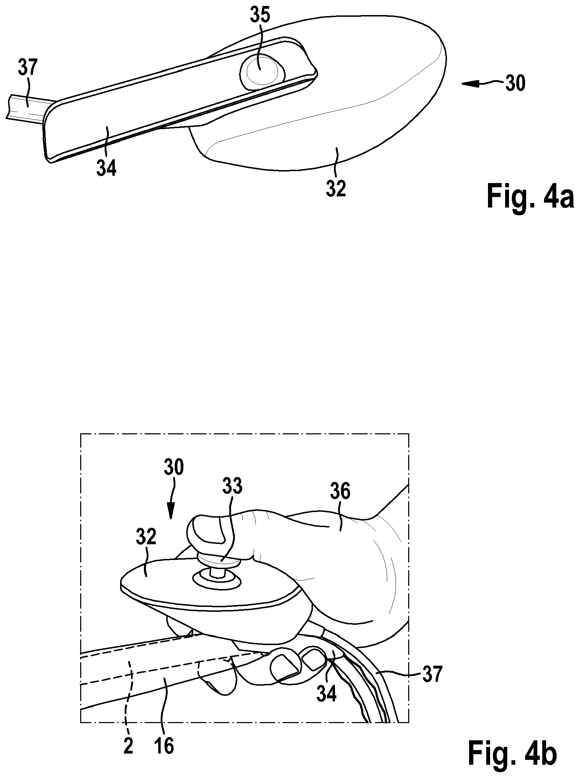

In Fig. 4a ist das Bedienteil 30 in einer perspektivischen Ansicht von der Unterseite her gesehen dargestellt. Die Halbschale 34 ist an der Unterseite des Trägers 32 befestigt. Durch eine Aussparung der Halbschale 34 ragt ein Drucksensor 35 in den von der Halbschale 34 gebildeten Raum hinein, so dass beim festen Anlegen des Schafts 2 der Drucksensor 35 betätigt wird. Wie in Fig. 4b gezeigt, kann das Bedienteil 30 gemeinsam mit dem Schaft 2 vom Bediener mit einer Hand 36 umgriffen werden und derart gehalten werden, dass sowohl eine Verschiebung des Schafts 2 gemeinsam mit dem Bedienteil 30 in einer Längsrichtung als auch ein Bedienen des Joysticks 33 mit einem Finger der Hand 36 möglich ist. Die Schaftschutzfolie 16 wird dabei mitergriffen. Der Joystick 33 dient zum Steuern der Abwinkelung des distalen Endbereichs des Schafts 2 und ist hierfür mit der nicht dargestellten Steuerungseinrichtung verbunden, die gemäß dem vom Joystick 33 abgegebenen Signal den Antrieb der Abwinkelung ansteuert. In der in den Figuren 4a und 4b gezeigten Ausführungsform ist das Bedienteil über ein Kabel 37 mit der Steuerungseinrichtung verbunden; es sind jedoch auch Varianten mit einer drahtlosen Signalübertragung möglich.

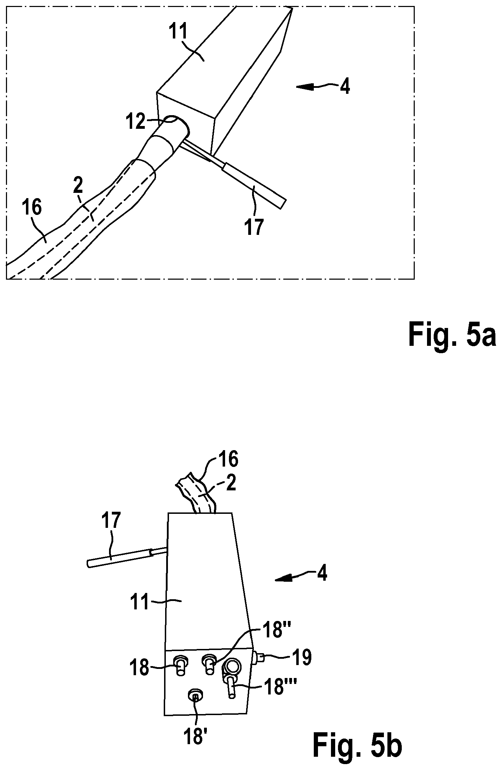

In den Figuren 5a und 5b ist die Endoskopbox 4 in zwei unterschiedlichen perspektivischen Ansichten dargestellt. Der Schaft 2 ist über das Drehlager 12 drehbar mit dem Gehäuse 11 der Endoskopbox 4 verbunden. Um die Ausführung der Rotation des Schafts zu erleichtern, ist ein Drehhebel 17 im Bereich des Drehlagers 12 am Schaft 2 angebracht. Die Schaftschutzfolie 16 ist vor dem Drehlager 12 mit Hilfe eines Klebebands am Schaft 2 befestigt und folgt somit einer Drehung des Schafts 2. Wie in Fig. 5b gezeigt ist, sind am proximalen Endbereich des Gehäuses 11 Anschlüsse 18, 18', 18" und 18'" für die Spül- und Saugfunktionen angeordnet, an denen entsprechenden Schläuche zur Verbindung mit externen Versorgungseinrichtungen angeschlossen werden können. Zur Reinigung und Sterilisation ist das Gehäuse 11 dicht ausgebildet, wobei die Anschlüsse 18, 18', 18", 18'" und die unten beschriebene Kupplung 19 sowie weitere Anschlüsse, die in Fig. 5a und 5b nicht dargestellt sind, durch eine Abdeckung dicht abgedeckt werden können.

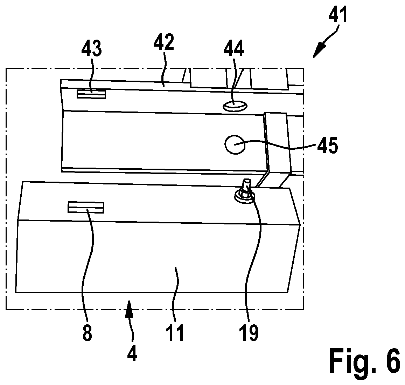

Wie in Fig. 6 gezeigt, ist ein Versorgungsadapter 41 einer externen Versorgungseinrichtung derart ausgebildet, dass das Gehäuse 11 der Endoskopbox 4 in eine als Winkel ausgebildete Aufnahme 42 derart eingesetzt werden kann, dass der Videoanschluss 8 mit einer Videobuchse 43 verbunden wird und eine Kupplung 19 mit einem Kupplungsanschluss 44 der Antriebe für die Seilzüge zur Abwinkelung des distalen Endbereichs des Schafts zusammenwirkt. Ferner ist in Fig. 6 der Lichtaustritt 45 zu erkennen, wobei aus Sicherheitsgründen das Licht erst im eingesetzten Zustand des Gehäuses 11 in die Aufnahme 42 freigegeben wird.

Zur Montage des erfindungsgemäßen Systems wird der Schaftstopper 20 an der Patientenliege 22 befestigt (s. Fig. 1). Der Schaft 2 des Endoskops 1 wird mit der Schaftschutzfolie 16 überzogen und in den Schaftstopper 20 auf die Gegendruckplatte 24 aufgelegt und im Schaftstopper 20 mit dem Bügel 25 befestigt bzw. durch die Haltebuchse 27 geschoben (s. Fig. 2a und 2b bzw. 3a und 3b). Die Druckplatte 23 wird über den Schaft geschwenkt, so dass der Schaft 2 zwischen der Druckplatte 23 und der Gegendruckplatte 24 verläuft. Die Schaftschutzfolie 16 wird bei der in Fig. 2a und 2b dargestellten Ausführungsform am Schlauchhalter 26 durch den Bügel 25 gesichert, wodurch auch der Schaft 2 selbst geführt wird; bei der in Fig. 3a und 3b gezeigten Ausführungsform wird der Schaft 2 mit der Schaftschutzfolie 16 durch die Haltebuchse 27 geführt und die Schaftschutzfolie 16 auf die Haltebuchse 27 aufgeklebt. Im Montagezustand des endoskopischen Systems ist der Schaft 2 in Längsrichtung verschiebbar. Die Endoskopbox 4 wird mit dem Gehäuse 11 in die Aufnahme 42 des Versorgungsadapters 41 der Versorgungseinrichtung eingesetzt (s. Fig. 6). Alternativ kann die Endoskopbox 4 auch durch den in Fig. 1 dargestellten Haltearm 14 gehalten werden, wobei die Verbindungsleitungen separat angeschlossen werden. Auch der Versorgungsadapter 41 kann von einem Haltearm gehalten werden.

Ist nun der Patient 40 auf der Patientenliege 22 in einem Abstand von etwa 15 - 20 cm zum Schaftstopper 20 gelagert, wie in Fig. 1 symbolisch dargestellt, so wird das System durch Einschalten in den Betriebszustand versetzt. In diesem Zustand ist der Schaft im Schaftstopper 20 gegen ein Verschieben blockiert, solange das Bedienteil 30 nicht auf den Schaft 2 aufgesetzt ist (s. Fig. 2a). Erst wenn der Bediener, der in der Regel der Gastroenterologe ist, der den endoskopischen Eingriff durchführt, das Bedienteil 30 auf den Schaft 2 aufsetzt und diesen fest in die Halbschale 34 hineindrückt, wird aufgrund des Signals des Drucksensors 35 von der Steuerungseinrichtung die bewegliche Druckplatte 23 derart angesteuert, dass der Schaft 2 freigegeben wird und gemeinsam mit der Schaftschutzfolie 16 innerhalb des Schaftstoppers 20 verschoben werden kann (s. Fig. 2b). Der Schaft 2 kann nun durch eine mit der Hand 36, die das Bedienteil 30 gemeinsam mit dem Schaft 2 umgreift, durchgeführte Schubbewegung in die Zugangsöffnung und durch diese in den körperinneren Hohlraum des Patienten 40 eingeführt werden. Auch eine Drehbewegung des Schafts 2 kann auf diese Weise durchgeführt werden, die zusätzlich durch den Drehhebel 17 unterstützt werden kann (s. Fig. 5a, 5b). Zur Erleichterung des Einführens kann der Chirurg durch Betätigung des Joysticks 33 nach Art einer Fernbedienung den abwinkelbaren distalen Endabschnitt 7 des Schafts 2 des Endoskops 1 motorisch steuern. Zusätzlich wird das Vorschieben des Schafts 2 dadurch vereinfacht, dass der Schaft 2 im Schaftstopper 20 geführt ist.

Während der Durchführung des endoskopischen Eingriffs befindet sich ein distaler Abschnitts des Schafts 2 innerhalb des endoskopischen Zugangswegs und des körperinneren Hohlraums des Patienten 40. Bei der Durchführung des Eingriffs kann der Schaft 2 vom Chirurgen durch Andrücken des Schafts 2 an das Bedienteil 30 mit derselben Hand 36 vor und zurück bewegt werden, und dann, wenn ein Halten in einer erreichten Längsposition gewünscht ist, kann durch Loslassen des Schafts 2 der Arretierungsmechanismus aktiviert und der Schaft 2 dadurch blockiert werden. Weitere Funktionen des endoskopischen Systems werden durch Betätigen weiterer Bedienelemente gesteuert, die am Bedienteil 30, an einem in den Figuren nicht dargestellten weiteren Bedienteil oder an der Steuerungseinrichtung angeordnet sein können, etwa Spül- und Saugfunktionen durch Steuerung entsprechender Ventile, die Funktion endoskopischer Arbeitsinstrumente durch Steuerung entsprechender motorischer Antriebe, oder die Aufnahme, Bearbeitung, Anzeige und Speicherung endoskopischer Aufnahmen durch Steuerung der Lichtquelle, der Videokamera und einer Bildverarbeitungs-, Anzeige- und Speicherungseinrichtung. Während der Durchführung des endoskopischen Eingriffs ist der außerhalb des Patienten 40 verbleibende Teil des Schafts 2 bis auf den Abschnitt distalseitig des Schlauchhalters 26 von der Schaftschutzfolie 16 umschlossen; dieser Abschnitt kommt nicht mit der Umgebung in Kontakt (s. Fig. 1).

Beim Herausziehen des Schafts 2 aus dem körperinneren Hohlraum des Patienten 40 legt sich automatisch die Schaftschutzfolie 16 um den durch den Schaftstopper 20 hindurchgeführten Teil des Schafts 2. Nach dem vollständigen Herausziehen des Schafts 2 kann die Schaftschutzfolie 16, die hierzu eine entsprechende Überlänge aufweist, über das distale Ende 5 des Schafts 2 gezogen werden, so dass der Schaft 2 nun vollständig von der Schaftschutzfolie eingeschlossen ist. Hierdurch wird der Transport des Endoskops 1 zur Reinigung erleichtert und eine Kontamination des Schaftstoppers 20, des Bedienteils 30 und der Patientenliege 22 vermieden.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Endoskop
- 2: Schaft
- 3: Proximales Ende
- 4: Endoskopbox
- 5: Distales Ende
- 6: Endoskopobjektiv
- 7: Distaler Endabschnitt
- 8: Videoanschluss
- 9: Öffnung
- 10: Arbeitskanalführung
- 11: Gehäuse
- 12: Drehlager
- 13: Antrieb
- 14: Haltearm
- 15: Verbindungsabschnitt
- 16: Schaftschutzfolie
- 17: Drehhebel
- 18, 18', 18", 18"': Anschlüsse
- 19: Kupplung
- 20: Schaftstopper
- 21: Halterung
- 22: Patientenliege
- 23: Druckplatte
- 23': Schwenkarm
- 24: Gegendruckplatte
- 25: Bügel
- 25': Gummiband
- 26: Schlauchhalter
- 27: Haltebuchse
- 28: Gabel
- 29: Kerbe
- 30: Bedienteil
- 31: Menüsteuerung
- 32: Träger
- 33: Joystick
- 34: Halbschale
- 35: Drucksensor
- 36: Hand
- 37: Kabel
- 40: Patient
- 41: Versorgungsadapter
- 42: Aufnahme
- 43: Videobuchse
- 44: Kupplungsanschluss
- 45: Lichtaustritt

## Patentansprüche

1. Endoskopisches System, insbesondere für die Koloskopie, umfassend ein flexibles Endoskop (1) mit einem langerstreckten flexiblen Schaft (2) und einem Kontrollteil sowie ein Bedienteil (30), das an den Schaft (2) ansetzbar oder auf diesen aufschiebbar ist und das mindestens ein Steuerelement aufweist, **dadurch gekennzeichnet, dass** das endoskopische System einen an einer Patientenunterlage befestigbaren Schaftstopper (20) umfasst., der einen durch das mindestens eine Steuerelement ansteuerbaren Arretierungsmcchanismus (23, 24) zum Arretierten des Schafts (2) gegen ein Verschieben in einer Längsrichtung des Schafts (2) aufweist, so dass in einem Betriebszustand des endoskopischen Systems der Arretierungsmechanismus durch das Steuerelement zum Arretieren und Freigeben des Schafts bezüglich einer Bewegung in Längsrichtung gesteuert wird, und dass das mindestens eine Steuerelement ein Schaftsensor (35) ist und dass der Arretierungsmechanismus durch den Selhaftsensor derart ansteuerbar ist, dass dann, wenn ein Signal des Schaftsensors anzeigt, dass das Bedienteil (30) nicht an den Schaft (2) angesetzt oder nicht auf diesen aufgeschoben ist, der Schaft (2) arretiert ist

2. Endoskopisches System nach Anspruch 1, **dadurch gekennzeichnet, dass** das Bedienteil (30) eine Halbschale (34) zur Aufnahme des Schafts (2) aufweist.

3. Endoskopisches System nach Anspruch 1 oder 2, **dadurch gekennzeichnet, dass** der Schaftsensor ein Drucksensor (35) ist.

4. Endoskopisches System nach einem der vorhergehenden Ansprüche, **dadurch gekennzeichnet, dass** das mindestens eine Steuerelement ein Bedienelement zum manuellen Steuern des Arretierungsmechanismus ist.

5. Endoskopisches System nach Anspruch 4, **dadurch gekennzeichnet, dass** der Schaftsensor, das Bedienelement und der Arretierungsmechanismus derart geschaltet sind, dass nur dann, wenn das Signal des Schaft-sensors anzeigt, dass das Bedienteil (30) an den Schaft (2) angesetzt oder auf diesen aufgeschoben ist, der Arretierungsmechanismus durch das Bedienelement steuerbar ist.

6. Endoskopisches System nach einem der vorhergehenden Ansprüche, **dadurch gekennzeichnet, dass** das Bedienteil (30) mindestens ein Bedienelement zur Steuerung einer Abwinkelung eines distalen Endabschnitts (7) des Schafts (2) aufweist,

7. Endoskopisches System nach einem der vorhergehenden Ansprüche, **dadurch gekennzeichnet, dass** das Bedienteil (30) zur Steuerung von endoskopischen Arbeitsinstrumenten ausgebildet ist oder ein weiteres Bedienteil zur Steuerung von endoskopischen Arbeitsinstrumenten vorgesehen ist.

8. Endoskopisches System nach einem der vorhergehenden Ansprüche, **dadurch gekennzeichnet, dass** der Arretierungsmechanismus über eine drahtlose Verbindung ansteuerbar ist.

9. Endoskopisches System nach einem der vorhergehenden Ansprüche, **dadurch gekennzeichnet, dass** der Schaftstopper (20) eine Durchgangsöffnung zum Halten des Schafts (2) aufweist.

10. Endoskopisches System nach einem der vorhergehenden Ansprüche, **dadurch gekennzeichnet, dass** der Schaftstopper (20) einen Schlauchhalter (26) aufweist zum Halten einer Schaftschutzfolie (16).

11. Endoskopisches System nach einem der vorhergehenden Ansprüche, **dadurch gekennzeichnet, dass** das Kontrollteil mindestens einen Anschluss (8, 18, 18', 18", 18"') zu Versorgung des Endoskops (1) und/oder mindestens eine Antriebskupplung (19) für Antriebe zur Abwinkelung eines distalen Endabschnitts (7) des Schafts (2) des Endoskops (1) umfasst.

12. Endoskopisches System nach einem der vorhergehenden Ansprüche, **dadurch gekennzeichnet, dass** der Schaft (2) um eine Längsachse des Schafts (2) drehbar mit dem Kontrollteil verbunden ist.

## Claims

1. Endoscopic system, in particular for colonoscopy, comprising a flexible endoscope (1) with an elongate and flexible shank (2), a regulator part, and also an operating part (30) which can be placed on or pushed onto the shank (2) and which has at least one control element, **characterized in that** the endoscopic system comprises a shank stop (20), which can be secured on a patient support and which has a locking mechanism (23, 24), controllable by the at least one control element, for locking the shank (2) against movement in a longitudinal direction of the shank (2), such that, in an operating state of the endoscopic system, the locking mechanism is controlled by the control element to lock and release the shank with respect to a movement in the longitudinal direction, and that the at least one control element is a shank sensor (35), and that the locking mechanism is controllable by the shank sensor in such a way that, when a signal of the shank sensor indicates that the operating part (30) is not placed on or pushed onto the shank (2), the shank (2) is locked.

2. Endoscopic system according to Claim 1, **characterized in that** the operating part (30) has a half-shell (34) for receiving the shank (2).

3. Endoscopic system according to Claim 1 or 2, **characterized in that** the shank sensor is a pressure sensor (35).

4. Endoscopic system according to one of the preceding claims, **characterized in that** the at least one control element is an operating element for manually controlling the locking mechanism.

5. Endoscopic system according to Claim 4, **characterized in that** the shank sensor, the operating element and the locking mechanism are connected in such a way that the locking mechanism is controllable by the operating element only when the signal of the shank sensor indicates that the operating part (30) is placed on or pushed onto the shank (2).

6. Endoscopic system according to one of the preceding claims, **characterized in that** the operating part (30) has at least one operating element for controlling an angle of a distal end portion (7) of the shank (2).

7. Endoscopic system according to one of the preceding claims, **characterized in that** the operating part (30) is designed to control endoscopic work instruments, or a further operating part is provided to control endoscopic work instruments.

8. Endoscopic system according to one of the preceding claims, **characterized in that** the locking mechanism is controllable via a wireless connection.

9. Endoscopic system according to one of the preceding claims, **characterized in that** the shank stop (20) has a through-opening for holding the shank (2).

10. Endoscopic system according to one of the preceding claims, **characterized in that** the shank stop (20) has a tube holder (26) for holding a shank protection film (16).

11. Endoscopic system according to one of the preceding claims, **characterized in that** the regulator part comprises at least one connection (8, 18, 18' 18", 18''') for supplying the endoscope (1), and/or at least one drive coupling (19) for drives that adjust the angle of a distal end portion (7) of the shank (2) of the endoscope (1).

12. Endoscopic system according to one of the preceding claims, **characterized in that** the shank (2) is connected to the regulator part so as to be rotatable about a longitudinal axis of the shank (2).

## Revendications

1. Système endoscopique, en particulier pour une coloscopie, comprenant un endoscope flexible (1) avec un câble flexible allongé (2) et une partie de contrôle ainsi qu'une partie de manoeuvre (30), qui peut être appliquée sur le câble (2) ou glissée sur celui-ci et qui présente au moins un élément de commande, **caractérisé en ce que** le système endoscopique comprend un arrêt de câble (20) pouvant être fixé à un support du patient, qui présente un mécanisme de blocage (23, 24) pouvant être commandé par ledit au moins un élément de commande pour bloquer le câble (2) contre un glissement dans une direction longitudinale du câble (2), de telle manière que dans un état de fonctionnement du système endoscopique le mécanisme de blocage soit commandé par l'élément de commande pour bloquer et libérer le câble à l'égard d'un mouvement en direction longitudinale, **en ce que** ledit au moins un élément de commande est une sonde de câble (35) et **en ce que** le mécanisme de blocage peut être commandé par la sonde de câble de telle manière que, lorsqu'un signal de la sonde de câble indique que la partie de manoeuvre (30) n'est pas montée sur le câble (2) ou n'est pas glissée sur celui-ci, le câble (2) soit bloqué.

2. Système endoscopique selon la revendication 1, **caractérisé en ce que** la partie de manoeuvre (30) présente une demi-coquille (34) pour le logement du câble (2).

3. Système endoscopique selon la revendication 1 ou 2, **caractérisé en ce que** la sonde de câble est un capteur de pression (35).

4. Système endoscopique selon l'une quelconque des revendications précédentes, **caractérisé en ce que** ledit au moins un élément de commande est un élément de manoeuvre pour la commande manuelle du mécanisme de blocage.

5. Système endoscopique selon la revendication 4, **caractérisé en ce que** la sonde de câble, l'élément de manoeuvre et le mécanisme de blocage peuvent être connectés de telle manière que le mécanisme de blocage ne puisse être commandé par l'élément de manoeuvre que lorsque le signal de la sonde de câble indique que la partie de manoeuvre (30) est montée sur le câble (2) ou est glissée sur celui-ci.

6. Système endoscopique selon l'une quelconque des revendications précédentes, **caractérisé en ce que** la partie de manoeuvre (30) présente au moins un élément de manoeuvre pour la commande d'un pliage d'une partie d'extrémité distale (7) du câble (2).

7. Système endoscopique selon l'une quelconque des revendications précédentes, **caractérisé en ce que** la partie de manoeuvre (30) est conçue pour la commande d'instruments de travail endoscopiques ou il est prévu une autre partie de manoeuvre pour la commande d'instruments de travail endoscopiques.

8. Système endoscopique selon l'une quelconque des revendications précédentes, **caractérisé en ce que** le mécanisme de blocage peut être activé par une liaison sans fil.

9. Système endoscopique selon l'une quelconque des revendications précédentes, **caractérisé en ce que** l'arrêt de câble (20) présente une ouverture de passage pour maintenir le câble (2).

10. Système endoscopique selon l'une quelconque des revendications précédentes, **caractérisé en ce que** l'arrêt de câble (20) présente un support de tuyau flexible (26) pour maintenir une feuille de protection du câble (16).

11. Système endoscopique selon l'une quelconque des revendications précédentes, **caractérisé en ce que** la partie de contrôle comprend au moins un raccord (8, 18, 18', 18", 18"') pour l'alimentation de l'endoscope (1) et/ou au moins un couplage d'entraînement (19) pour des entraînements destinés au pliage d'une partie d'extrémité distale (7) du câble (2) de l'endoscope (1).

12. Système endoscopique selon l'une quelconque des revendications précédentes, **caractérisé en ce que** le câble (2) est relié à la partie de contrôle d'une façon rotative autour d'un axe longitudinal du câble (2).
